# EUROPÄISCHE PATENTSCHRIFT

(11) **EP 0 772 589 B1**
(45) Veröffentlichungstag und Bekanntmachung des Hinweises auf die Patenterteilung: **24.03.1999**
(21) Anmeldenummer: 95929017.2
(22) Anmeldetag: 27.07.1995
(51) Int. Cl.: C07C 409/04

(54) **VERFAHREN ZUR HERSTELLUNG VON 2,5-DIMETHYL-2,5-DIHYDROPEROXY-HEXAN**
PROCESS FOR PRODUCING 2,5-DIMETHYL-2,5-DIHYDROPEROXYHEXANE
PROCEDE DE PRODUCTION DE 2,5-DIMETHYL-2,5-DIHYDROPEROXYHEXANE

(30) Priorität: 28.07.1994 DE 4426839
(43) Veröffentlichungstag der Anmeldung: 14.05.1997
(73) Patentinhaber: Peroxid-Chemie GmbH, 82049 Pullach (DE)
(72) Erfinder: HÄGEL, Eberhard, D-82057 Icking (DE); ZEISS, Werner, D-82547 Eurasburg (DE)
(74) Vertreter: Böhm, Brigitte, Dipl.-Chem. Dr.
(86) Internationale Anmeldenummer: EP9502987
(87) Internationale Veröffentlichungsnummer: WO9603372

(56) Entgegenhaltungen:
- US-A- 4 154 768
- US-A- 5 149 883

## Beschreibung

Die Erfindung betrifft ein Verfahren zur Herstellung von 2,5-Dimethyl-2,5-dihydroperoxy-hexan durch Umsetzung von 2,5-Dimethyl-1,5-hexadien mit Wasserstoffperoxid in einem sauren Medium.

2,5-Dimethyl-2,5-dihydroperoxy-hexan (DHHP) ist ein wichtiges Ausgangsprodukt zur Herstellung von Vernetzungsperoxiden (Dialkylperoxide, wie z.B. 2,5-Dimethyl-2,5-di-tert.-butyl-peroxy-hexan, oder Perketale, wie 3,3,6,6,9, 9-Hexamethyl-1,2,4,5-tetraoxa-cyclononan) (vgl. z.B. GB-PS-936008), sowie von hochaktiven, bifunktionellen Perestern.

In der FR-PS-1291965 wird zwar auch die Herstellung von 2,5-Dimethyl-2,5-di-tert.-butylperoxy-hexan aus 2,5-Dimethyl-1,5-hexadien durch Umsetzung mit tert.-Butylhydroperoxid beschrieben; die dabei erhaltene Ausbeute von 26 % ist aber sehr unbefriedigend, und außerdem sind nach diesem Verfahren nur Dialkylperoxide, aber keine Perketal- und Perester-Derivate von DHHP zugänglich.

Als Verfahren zur Herstellung von 2,5-Dimethyl-2,5-dihydroperoxy-hexan (DHHP) ist aus dem Stand der Technik die Umsetzung von 2,5-Dimethylhexan mit Sauerstoff bekannt.

Die EP-A-0513711 beschreibt ein Verfahren zur Herstellung aliphatischer Hydroperoxide, z.B. von 2,5-Dimethyl-2,5-dihydroperoxy-hexan (DHHP) durch Umsetzung eines entsprechenden Tetrahydrofuranderivats mit Wasserstoffperoxid.

Die US-A-4154768 beschreibt ein allgemeines Verfahren zur Herstellung von tert.-Alkylhydroperoxiden aus asymetrischen sekundären Olefinen durch Umsetzung mit Wasserstoffperoxid in einer eine starke Säure enthaltenden Reaktionsmischung.

Nach JP-A-3215468 und JP-A-3190856 werden Hydroperoxide durch Umsetzung eines Olefins oder Alkohols mit Wasserstoffperoxid in Gegenwart einer starken Säure erhalten.

Üblicherweise erfolgt die Herstellung von 2,5-Dimethyl-2,5-dihydroperoxy-hexan (DHHP) durch Umsetzung von 2,5-Dimethylhexan-2,5-diol mit Wasserstoffperoxid in Gegenwart eines sauren Katalysators, im allgemeinen unter Verwendung von Schwefelsäure (vgl. Rust, J.Am.Chem. Soc. 79 (1957) 4000, 4002; L. Criegee, Erdöl und Kohle 15 (1962) 523, 524, 528; Criegee und Paulig, Chem. Ber. 88 (1955), 712, 716; Criegee und Dietrich, Liebigs Ann. Chem 560 (1948) 141,; L. Dulog und A. Sanner, Tetrahedron Letters 51 (1966) 6353-6358). Nach diesem Verfahren erfolgt die Dosierung des Diols als Feststoff, was einen Nachteil darstellt. Ein weiterer Nachteil dieses Verfahrens ist auch die leichte Bildung von cyclisierungsprodukten (z.B. 2,2,5,5-Tetramethyl-tetrahydrofuran) aus dem Diol unter Säureeinfluß, was zu schlechten Ausbeuten an Dihydroperoxid und einem verunreinigten Produkt führt.

Es bestand daher weiterhin ein hohes Bedürfnis an einem Verfahren zur Herstellung von 2,5-Dimethyl-2,5-dihydroperoxy-hexan (DHHP), das auf einfache und technisch vorteilhafte Weise durchgeführt werden kann, und mit dem das DHHP mit hoher Ausbeute erhältlich ist.

Diese Aufgabenstellung wird mit dem Verfahren der vorliegenden Erfindung gelöst.

Gegenstand der vorliegenden Erfindung ist ein Verfahren zur Herstellung von 2,5-Dimethyl-2,5-dihydroperoxy-hexan (DHHP) gemäß Patentanspruch 1 durch Umsetzung von 2,5-Dimethyl-1,5-hexadien mit Wasserstoffperoxid in einem sauren Medium.

2,5-Dimethyl-1,5-hexadien reagiert als Olefin mit einer wäßrigen Mischung von Wasserstoffperoxid und Schwefelsäure bei üblichen Reaktionstemperaturen kaum. Durch Zugabe einer kleinen Menge eines geeigneten Emulgators oder eines mit Wasser mischbaren Lösungsmittels tritt jedoch eine rasche Reaktion zu DHHP ein.

Vorzugsweise wird daher die Umsetzung in einem sauren wäßrigen Medium unter Zusatz eines Emulgators oder eines wassermischbaren Lösemittels durchgeführt.

Als wassermischbare Lösemittel können im Rahmen der Erfindung beispielsweise verwendet werden: Triethylphosphat, Tetrahydrofuran, 1,4-Dioxan, Glykole oder Glykolether, wovon Triethylphosphat besonders bevorzugt ist.

Besonders geeignete Emulgatoren sind nichtionische Oxethylate von aliphatischen Fettalkoholen oder Oxoalkoholen mit 10 bis 18 C-Atomen, wie C₁₃/C₁₅-Oxoalkohole mit 8 bis 10 Mol Ethylenoxid; C₁₀-Oxoalkohol mit 6 bis 7 Mol Ethylenoxid; C₁₃-Oxoalkohol mit 8 Mol Ethylenoxid, C₁₀/C₁₂-Fettalkohol-propoxyliert/ethoxyliert und Sorbitanester-oxethylate.

Die Umsetzung erfolgt vorzugsweise bei einer Temperatur im Bereich von 0 °C bis 60 °C, und insbesondere im Bereich von 10 bis 50 °C, wobei aber auch, insbesondere abhängig vom verwendeten Reaktionsmedium und/oder der verwendeten Säure, Temperaturen oberhalb oder unterhalb dieser Bereiche möglich sind.

Als saurer Katalysator wird vorzugsweise eine Säure verwendet, die ausgewählt ist aus der Gruppe bestehend aus Schwefelsäure, Phosphorsäure, Alkylsulfonsäure, optional substituierte Arylsulfonsäuren, Polyphosphorsäure und Perchlorsäure, wobei Schwefelsäure insbesondere bevorzugt ist.

In einer besonders bevorzugten Ausführungsform des erfindungsgemäßen Verfahrens wird das 2,5-Dimethyl-1,5-hexadien zu einem wässerigen Gemisch aus Wasserstoffperoxid und Schwefelsäure und Emulgator unter Rühren zugegeben.

Die Wasserstoffperoxidkonzentration im Reaktionsmedium beträgt vorzugsweise 20 bis 50 Gew.-%, und insbesondere 30 bis 50 Gew.-%; das Wasserstoffperoxid wird vorzugsweise in einem molaren Überschuß eingesetzt, und insbesondere in einem Verhältnis von 1 Mol 2,5-Dimethyl-1,5-hexadien zu 3 bis 8 Mol, und in erster Linie 5 Mol, Wasserstoffperoxid.

Die nach dem erfindungsgemäßen Verfahren erhältliche Ausbeute an 2,5-Dimethyl-2,5-dihydroperoxy-hexan (DHHP) ist sehr gut, und beträgt in der Regel mindestens 55 % (bezogen auf 2,5-Dimethyl-1,5-hexadien). Durch Verwendung von 2,5-Dimethyl-1,5-hexadien wird außerdem auch ein Ausgangsmaterial eingesetzt, das unter den Verfahrensbedingungen eine Flüssigkeit ist, wodurch eine technisch aufwendige Feststoffdosierung, wie sie bei dem Verfahren zur Herstellung von DHHP durch Umsetzung von 2,5-Dimethyl-hexan-2,5-diol mit Wasserstoffperoxid erforderlich ist, vermieden werden kann.

Die nachfolgenden Beispiele sollen die Erfindung näher erläutern, ohne sie darauf zu beschränken.

### Beispiel 1

Zu einer Mischung aus 194 g 70 %-Wasserstoffperoxid (4,0 Mol), 150 g 72 %-Schwefelsäure (1,1 Mol) und 25 g Triethylphosphat werden unter Rühren und Kühlen 113 g 2,5-Dimethyl-1,5-hexadien (1,0 Mol) in ca. 50 Minuten so zugegeben, daß die Temperatur bei 30°C gehalten wird. Bereits nach wenigen Minuten beginnt das DHHP zu kristallisieren. Nach beendeter Zugabe wird noch 2 Stunden bei 30°C gerührt.
Die Reaktionsmischung wird mit 100 ml Wasser verdünnt, filtriert und das DHHP mit kaltem Wasser gewaschen. Man erhält 112,4 g wasserfeuchtes DHHP mit einem Gehalt von 80,4 % (entsprechend 50,7 % der theoretischen Ausbeute).

### Beispiel 2

Zu einer Mischung von 329 g 70 %-Wasserstoffperoxid (8,0 Mol) und 218 g 72 %-Schwefelsäure (1,6 Mol) werden 4 g Tween 21 (Sorbitanmonolaurat-4 EO) gegeben. Bei 25°C tropft man unter Rühren und Kühlen 226 g 2,5-Dimethyl-1,5-hexadien (2,0 Mol) so zu, daß die Temperatur bis maximal 35°C steigt. Nach wenigen Minuten beginnt das DHHP zu kristallisieren. Nach beendeter Zugabe rührt man noch 2 Stunden bei 30°C, verdünnt die Reaktionsmischung mit 250 ml Wasser, filtriert das DHHP ab und wäscht es mit kaltem Wasser frei von Wasserstoffperoxid. Man erhält 310 g wasserfeuchtes DHHP mit einem Gehalt von 73,4 % (entsprechend 63,8 % der theoretischen Ausbeute).

### Beispiel 3

Man verfährt wie in Beispiel 2, gibt aber zu 301 g 68 %-Wasserstoffperoxid (6,0 Mol) und 214 g 65 %-Schwefelsäure (1,42 Mol) 3 g Lutensol TO 8 (C₁₃-Oxoalkohol mit 8 Mol Ethylenoxid) und tropft 170 g 2,5-Dimethyl-1,5-hexadien (1,5 Mol) zu. Man erhält 252 g wasserfeuchtes DHHP mit einem Gehalt von 76,2 % (entsprechend 71,8 % der theoretischen Ausbeute).

### Beispiel 4

Man verfährt wie in Beispiel 2, gibt aber zu 315 g 68 %-Wasserstoffperoxid (6,3 Mol) und 210 g 65 %-Schwefelsäure (1,39 Mol) 2 g Marlox FK 86 E (C₁₀/C₁₂-Fettalkohol-propoxylier/ethoxyliert) und tropft 113 g 2,5-Dimethyl-1,5-hexadien (1,0 Mol) zu.
Man erhält 182 g wasserfeuchtes DHHP mit einem Gehalt von 73,1 % (entsprechend 74,6% der theoretischen Ausbeute).

## Patentansprüche

1. Verfahren zur Herstellung von 2,5-Dimethyl-2,5-dihydroperoxy-hexan durch Umsetzung von 2,5-Dimethyl-1,5-hexadien mit Wasserstoffperoxid in einem sauren Medium.

2. Verfahren nach Anspruch 1,
**dadurch gekennzeichnet**,
daß man die Umsetzung in einem sauren wässerigen Medium unter Zusatz eines Emulgators durchführt.

3. Verfahren nach Anspruch 2,
**dadurch gekennzeichnet**,
daß man als Emulgator ein nichtionisches Oxethylat eines Fettalkohols oder Oxoalkohols mit 10 bis 18 C-Atomen verwendet.

4. Verfahren nach Anspruch 2 oder 3,
**dadurch gekennzeichnet**,
daß man das 2,5-Dimethyl-1,5-hexadien zu einem wäßrigen Gemisch aus Wasserstoffperoxid, Schwefelsäure und Emulgator unter Rühren zugibt.

5. Verfahren nach Anspruch 1,
**dadurch gekennzeichnet**,
daß man die Umsetzung unter Zusatz eines wassermischbaren Lösemittels durchführt.

6. Verfahren nach Anspruch 5,
**dadurch gekennzeichnet**,
daß man als Lösemittel Triethylphosphat verwendet.

7. Verfahren nach Anspruch 1 oder 2,
**dadurch gekennzeichnet**,
daß man die Mischung bei einer Temperatur im Bereich von 0°C bis 60°C und insbesondere im Bereich von 10 bis 50°C durchführt.

8. Verfahren nach einem der Ansprüche 1 bis 3,
**dadurch gekennzeichnet**,
daß man als saures wässeriges Medium wässerige Schwefelsäure verwendet.

## Claims

1. Process for the preparation of 2,5-dimethyl-2,5-dihydroperoxyhexane by reaction of 2,5-dimethyl-1,5-hexadiene with hydrogen peroxide in an acidic medium.

2. Process according to claim 1**,** characterised in that one carries out the reaction in an acidic aqueous medium with addition of an emulsifier.

3. Process according to claim 2, characterised in that one uses a non-ionic oxyethylate of a fatty alcohol or oxoalcohol with 10 to 18 C-atoms as emulsifier.

4. Process according to claim 2 or 3, characterised in that one adds the 2,5-dimethyl-1,5-hexadiene, while stirring, to an aqueous mixture of hydrogen peroxide, sulphuric acid and emulsifier.

5. Process according to claim 1, characterised in that one carries out the reaction with addition of a water-miscible solvent.

6. Process according to claim 5, characterised in that one uses triethyl phosphate as solvent.

7. Process according to claim 1 or 2, characterised in that one carries out the mixing at a temperature in the range of 0°C to 60°C and especially in the range of 10 to 50°C.

8. Process according to one of claims 1 to 3, characterised in that one uses aqueous sulphuric acid as acidic aqueous medium.

## Revendications

1. Procédé pour la préparation de 2,5-Diméthyl-2,5-dihydroperoxy-hexane par mise en réaction de 2,5-Diméthyl-1,5-hexadiène avec de l'eau oxygénée ou peroxyde d'hydrogène dans un milieu acide.

2. Procédé selon la revendication 1, caractérisé en ce que la réaction est conduite dans un milieu aqueux acide avec addition d'un agent d'émulsion.

3. Procédé selon la revendication 2, caractérisé en qu'en tant qu'agent d'émulsion on utilise un oxéthylate non ionique d'un alcool gras ou d'un oxoalcool avec 10 à 18 atomes C.

4. Procédé selon la revendication 2 ou 3, caractérisé en ce que l'on ajoute le 2-5-Diméthyl-1,5-hexadiène à un mélange aqueux constitué d'eau oxygénée ou peroxyde d'hydrogène, d'acide sulfurique et d'un agent d'émulsion sous agitation.

5. Procédé selon la revendication 1, caractérisé en ce que l'on conduit la réaction en ajoutant un solvant miscible à l'eau.

6. Procédé selon la revendication 5, caractérisé en ce qu'en tant que solvant, on utilise du triéthylphosphate.

7. Procédé selon la revendication 1 ou 2, caractérisé en ce que l'on conduit le mélange à une température dans la plage de 0 à 60°C et en particulier dans la plage de 10 à 50°C.

8. Procédé selon une des revendications 1 à 3, caractérisé en ce qu'en tant que milieu aqueux acide, on utilise de l'acide sulfurique aqueux.
